(19) Europäisches Patentamt European Patent Office Office européen des brevets

(11) **EP 3 346 931 B1**

(12) **FASCICULE DE BREVET EUROPEEN**

(45) Date de publication et mention de la délivrance du brevet:
**01.05.2019 Bulletin 2019/18**

(21) Numéro de dépôt: **16766025.7**

(22) Date de dépôt: **22.08.2016**

(51) Int Cl.:
***A61B 17/29*** *(2006.01)*

(86) Numéro de dépôt international:
**PCT/FR2016/000131**

(87) Numéro de publication internationale:
**WO 2017/042441 (16.03.2017 Gazette 2017/11)**

(54) **PINCE DE COELIOSCOPIE**

LAPAROSKOPISCHE ZANGE

LAPAROSCOPY FORCEPS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.09.2015 FR 1501851**

(43) Date de publication de la demande:
**18.07.2018 Bulletin 2018/29**

(73) Titulaire: **AB Medica**
**18100 Mery-sur-Cher (FR)**

(72) Inventeur: **BLANC Alexandre**
**13013 Marseille (FR)**

(74) Mandataire: **Flavenot, Bernard**
**ABRITT**
**17, rue du Docteur Charcot**
**91290 La Norville (FR)**

(56) Documents cités:
**EP-A1- 2 147 638      DE-A1-102011 001 890**
**DE-U1- 29 911 011     JP-A- H06 296 619**
**US-A- 5 312 434       US-A1- 2010 249 700**
**US-A1- 2012 203 063**

EP 3 346 931 B1

**Description**

La présente invention concerne les pinces de coelioscopie.

La coelioscopie, parfois désignée «laparoscopie», est une technique chirurgicale mini-invasive d'intervention et de diagnostic dans notamment les cavités humaines abdominales comme l'appareil digestif, mais aussi en gynécologie et en urologie. Elle se pratique au moyen d'une pince dite "pince de coelioscopie".

Il est déjà connu de telles pinces de coelioscopie dont une vue générale et schématique est illustrée sur la figure 1, par exemple celles décrites et illustrées dans les documents US 5 312 434 et DE 299 11 011 U1.

Une telle pince comporte une chape comprenant une percée traversante cylindrique de révolution définie entre une extrémité proximale et une extrémité distale, deux mors à faces de préhension, des moyens pour monter en rotation, à l'extrémité distale de la percée traversante, les deux mors par rapport à la chape autour d'un premier axe de façon que ces deux mors soient aptes à prendre toutes positions entre deux positions extrêmes, à savoir une position ouverte quand les deux faces de préhension respectivement des deux mors font entre elles un angle non nul et une position fermée quand les deux faces de préhension respectivement des deux mors font entre elles un angle sensiblement nul.

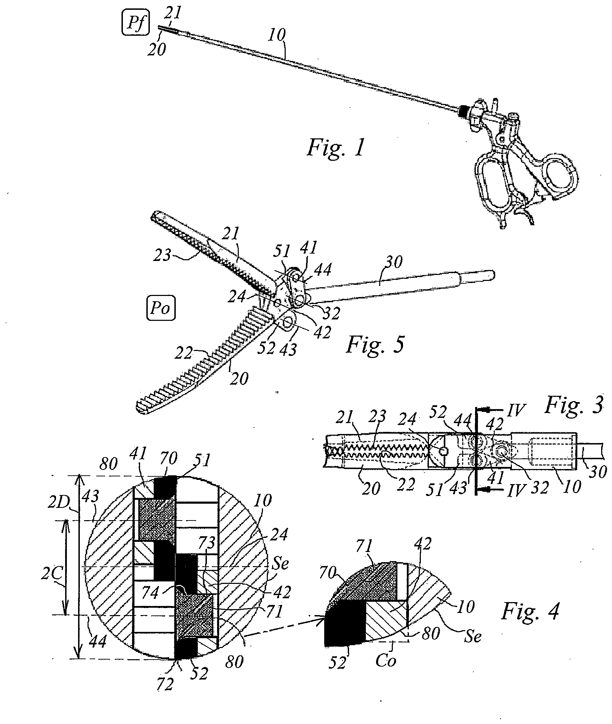

La pince comprend en outre une tige de commande montée en translation dans la percée traversante et définie entre une extrémité proximale et une extrémité distale, l'extrémité proximale étant reliée à des moyens de commande de la translation de la tige dans la chape, par exemple du type à gâchette ou analogue comme illustré sur la figure 1.

Pour transformer le mouvement de translation de la tige de commande en des mouvements de rotation des mors, la pince comporte des moyens formant pantographe qui relient l'extrémité distale de la tige de commande respectivement aux deux mors.

Il est aussi connu que de tels moyens formant pantographe comprennent essentiellement deux biellettes, des moyens pour monter en rotation les deux biellettes, respectivement par une première de leurs deux extrémités, à l'extrémité distale de la tige, par rapport à la tige de commande et autour d'un deuxième axe, deux cames respectivement solidaires des deux mors par une première de leurs deux extrémités, les axes longitudinaux de ces deux cames formant entre eux un angle sensiblement nul quand les deux faces de préhension des mors sont au contact, des moyens pour monter en rotation la seconde extrémité de chaque biellette sur la seconde extrémité des deux cames autour respectivement d'un troisième axe et d'un quatrième axe, les premier, deuxième, troisième et quatrième axes étant définis de façon à être parallèles.

Une telle pince de coelioscopie donne de bons résultats, mais peut présenter des inconvénients.

Notamment, les pinces selon le US 5 312 434 et le DE 299 11 011 U1 présentent une section hors tout qui est plus grande que le diamètre extérieur de la chape, ce qui entraîne l'inconvénient de prévoir une tube guide dans lequel est enfichée la chape d'un diamètre intérieur plus grand que le diamètre extérieur de la chape. Dans ce cas, la chape n'est donc pas parfaitement guidée et la pince peut subir des déplacements latéraux qui nuisent à la précision de l'intervention.

En effet, comme les biellettes sont soumises à des forces relativement importantes lorsque la pince est utilisée, il arrive qu'elles se rompent relativement souvent, nuisant de ce fait à la fiabilité de la pince.

Aussi, la présente invention a-t-elle pour but de réaliser une pince pour coelioscopie qui pallie les inconvénients mentionnés ci-dessus, sans pour autant être plus onéreuse que les pinces pour coelioscopie de l'art antérieur, qui ait une structure répondant aux deux conditions antagonistes suivantes : présenter un encombrement réduit pour pouvoir être introduite dans les cavités humaines via un tube guide qui est de fait limité en diamètre de passage, et qui soit d'une solidité maximale pour réaliser l'opération souhaitée, par exemple l'ablation d'un élément de corps malade, en évitant une rupture in situ d'un des éléments constitutifs de la pince.

Plus précisément, la présente invention a pour objet une pince de coelioscopie comportant au moins :

- une chape ayant un diamètre extérieur constant sur toute sa longueur fonctionnelle définie entre son extrémité proximale et son extrémité distale, et comprenant une percée traversante cylindrique de révolution définie entre ses extrémités proximale et distale,
- deux mors à faces de préhension,
- des moyens pour monter en rotation, à l'extrémité distale de ladite percée traversante, les deux mors par rapport à ladite chape autour d'un premier axe de façon que ces deux mors soient aptes à prendre toutes positions entre deux positions extrêmes, respectivement une position ouverte quand les deux faces de préhension respectivement des deux mors font entre elles un angle non nul et une position fermée quand les deux faces de préhension respectivement des deux mors font entre elles un angle sensiblement nul,
- une tige de commande montée en translation dans ladite percée traversante et définie entre une extrémité proximale et une extrémité distale,
- des moyens formant pantographe pour lier l'extrémité distale de ladite tige de commande respectivement aux deux dits mors, lesdits moyens formant pantographe comprenant :

* deux biellettes,
* des moyens pour monter en rotation les deux dites biellettes, respectivement par une première de leurs deux extrémités, à l'extrémité distale de ladite tige, par rapport à la tige de commande et autour d'un deuxième axe,
* deux cames respectivement solidaires des deux mors par une première de leurs deux extrémités,
* des moyens pour monter en rotation la seconde extrémité de chaque biellette sur la seconde extrémité de chaque came autour respectivement d'un troisième axe et d'un quatrième axe, lesdits premier, deuxième, troisième et quatrième axes étant définis de façon à être parallèles,
caractérisée par le fait que :

- le diamètre extérieur de ladite chape ayant une valeur égale à 2D,
- les biellettes étant identiques et formées chacune d'une plaque à section transversale rectangulaire à bout arrondi, la valeur de la largeur de cette plaque étant égale à 2E,
- la distance séparant les troisième et quatrième axes de rotation quand les deux faces de préhension des mors sont au contact ayant une valeur 2C inférieure à 2D, et
- la valeur de l'angle que font les axes des deux biellettes quand les deux faces de préhension des mors sont au contact, étant égale à 2A,
les valeurs D, E, C et A sont sensiblement liées par l'équation suivante :

$$D = C + E/cosinus\ A,$$

et que
- le bord extérieur de la seconde extrémité de chaque biellette comporte une partie sensiblement cylindrique chanfreinée d'un rayon sensiblement égal à celui de la surface extérieure de ladite chape de façon que, quand les deux mors sont en position fermée, lesdits moyens formant pantographe soient inscrits dans et sur la surface enveloppante définie par ladite surface extérieure de ladite chape.

D'autres caractéristiques et avantages de l'invention apparaîtront au cours de la description suivante donnée en regard des dessins annexés à titre illustratif mais nullement limitatif, dans lesquels :

La figure 1 est une vue d'ensemble d'une pince de coelioscopie aussi bien selon l'art antérieur que selon l'invention,
La figure 2 est une vue de principe schématique partiellement en écorché d'une partie de pince de coelioscopie en accord avec la présente invention,
Les figures 3 et 4 représentent respectivement, pour la figure 3 : une vue en coupe longitudinale schématique de la partie de la pince de coelioscopie industrielle selon l'invention en accord avec la figure 2 qui est une représentation de cette partie à plus grande échelle que sur cette figure 3 et, pour la figure 4 : une vue en coupe transversale référencée IV-IV sur la figure 3 avec en plus un effet de loupe sur la portion entourée de cette figure 4, de façon à bien faire ressortir une caractéristique de l'invention.
Les figures 5, 6 et 7 représentent, en accord avec les figures 1 à 4, trois vues différentes en perspective cavalière permettant de bien comprendre la structure de la pince de coelioscopie selon l'invention.

Il est tout d'abord précisé que, dans la présente description, si l'adverbe "sensiblement" est associé à un qualificatif d'un moyen donné, sans autre précision particulière, ce qualificatif doit être compris au sens strict ou approché.
La présente invention concerne, en référence à toutes les figures, une pince de coelioscopie comportant au moins une chape 10 ayant un diamètre extérieur constant sur toute sa longueur fonctionnelle définie entre son extrémité proximale 12 et son extrémité distale 13, et comprenant en outre une percée traversante cylindrique de révolution 11 définie entre son extrémité proximale 12 et son extrémité distale 13, une telle chape étant généralement et très avantageusement constituée d'un tube cylindrique de révolution.
La pince comporte aussi deux mors 20, 21 à faces de préhension 22, 23, éventuellement dentées comme illustré.
Par « face de préhension », il est entendu, au sens de la présente description, toute surface apte à venir au contact d'un corps donné pour, par exemple, le saisir, l'écraser, le couper, l'amputer, etc..
La pince comporte en outre des moyens pour monter en rotation, à l'extrémité distale 13 de la percée traversante 11, les deux mors par rapport à la chape autour d'un premier axe 24 de façon que ces deux mors soient aptes à prendre toutes positions entre deux positions extrêmes, à savoir une position ouverte Po quand les deux faces de préhension respectivement des deux mors font entre elles un angle non nul et une position fermée Pf, figures 1, 2 et 3, quand les deux faces de préhension respectivement des deux mors font entre elles un angle sensiblement nul.
Il est en outre prévu une tige de commande 30 montée en translation Tr dans la percée traversante 11 et définie entre une extrémité proximale 35 et une extrémité distale 31, l'extrémité proximale de cette tige de commande 30 étant couplée à des moyens du type gâchette ou analogue pour commander la translation de la tige de commande dans la chape dans les deux sens. Ces derniers moyens sont bien connus en eux-mêmes et ne seront pas plus amplement décrits ici par unique souci de simplifier la présente description.

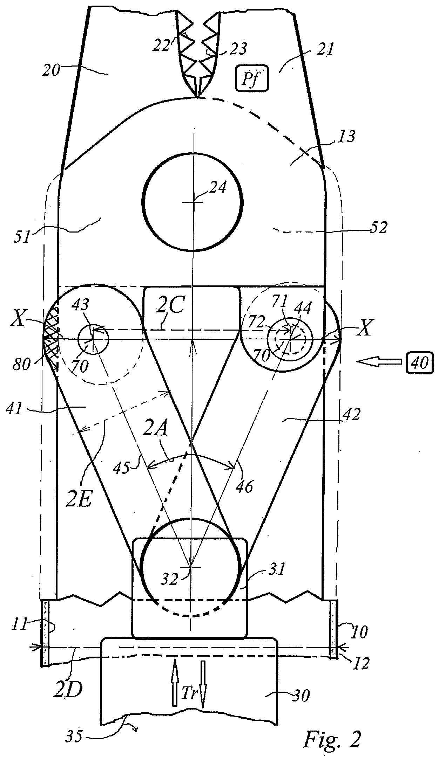

Sont aussi prévus des moyens formant pantographe 40 pour lier l'extrémité distale 31 de la tige de commande respectivement aux deux mors, comme plus particulièrement visible sur la figure 2. Ces moyens formant pantographe comprennent, de façon connue, deux biellettes 41, 42, des moyens pour monter en rotation les deux biellettes, respectivement par une première de leurs deux extrémités, à l'extrémité distale 31 de la tige 30, par rapport à la tige de commande et autour d'un deuxième axe 32, et deux cames 51, 52 respectivement solidaires des deux mors 20, 21 par une première de leurs deux extrémités.

Ces moyens de pantographe comprennent en outre des moyens pour monter en rotation la seconde extrémité de chaque biellette 41, 42 sur la seconde extrémité de chaque came 51, 52 autour respectivement d'un troisième axe 43 et d'un quatrième axe 44.

Il est en outre défini que ces premier 24, deuxième 32, troisième 43 et quatrième 44 axes sont tous parallèles entre eux.

Selon une caractéristique essentielle de l'invention, sachant :

* que le diamètre extérieur de la chape 10 a une valeur égale à 2D,
* que, les biellettes 41, 42 étant identiques et formées chacune d'une plaque à section transversale rectangulaire à bout arrondi ou équivalent, voir à titre d'exemple la figure 2, la valeur de la largeur de cette plaque est égale à 2E,
* que la distance séparant les troisième 43 et quatrième 44 axes de rotation quand les deux faces de préhension 22, 23 des mors 20, 21 sont au contact a une valeur 2C inférieure à 2D, et
* que la valeur de l'angle que font les axes 45, 46 des deux biellettes 41, 42 quand les deux faces de préhension des mors sont au contact est égale à 2A,
les valeurs D, E, C et A sont sensiblement liées par l'équation suivante :

$$D = C + E/\text{cosinus } A.$$

La valeur cosinus A est déterminée par le rapport Z/Y où :

- Y est la valeur de la distance entre le deuxième axe 32 et l'un des troisième 43 ou quatrième axe 44, et
- Z est la valeur de la distance entre ce premier axe 32 et là droite passant par les troisième 43 et quatrième 44 axes, quand les faces de préhension 22, 23 sont au contact.

Il est précisé que les termes "sensiblement liées par l'équation suivante" signifient que la valeur "C + E/cosinus A" peut être strictement égale à D ou très légèrement inférieure, à cause notamment de la forme "arrondie" des extrémités des biellettes.

En outre, comme les biellettes sont réalisées dans des plaques à section rectangulaire, l'un de leur coin Co, celui correspondant au bord extérieur de leur seconde extrémité comme illustré dans la partie de figure 4 avec effet de loupe, dépasserait de la surface extérieure Se de la chape 10 et ne permettrait pas d'introduire la pince dans le tube guide.

Aussi, pour que la pince puisse être glissée dans le tube guide, le bord extérieur de la seconde extrémité de chaque biellette comporte une partie sensiblement cylindrique chanfreinée 80 d'un rayon sensiblement égal à celui de la surface extérieure Se de la chape 10 de façon que, quand les deux mors 20, 21 sont en position fermée Pf, les moyens formant pantographe 40 soient inscrits dans et sur la surface enveloppante définie par cette surface Se.

Ainsi, cette partie chanfreinée 80 peut sans difficulté glisser sur la surface intérieure du tube guide quand la pince est introduite dans ce tube guide.

La réalisation de ce chanfrein est tout à fait possible et n'affaiblit que très peu la biellette, et en aucune façon l'assemblage des biellettes et des cames par les pions 70 comme décrit ci-après.

Selon une autre caractéristique importante et préférentielle de l'invention, les deux cames 51, 52 sont situées entre les deux biellettes 41, 42.

En outre, les moyens pour monter en rotation la seconde extrémité d'une biellette 41, 42 sur la seconde extrémité d'une came 51, 52 sont constitués par un pion 70 comportant une tige de pion 71 et une tête d'épaulement 72 solidaire de la tige de pion 71, deux orifices cylindriques de révolution 73, 74 réalisés de façon coaxiale respectivement dans la biellette et dans la came, la section transversale de ces orifices étant sensiblement complémentaires de la section transversale de la tige de pion 71 de façon que cette dernière soit apte à être insérée de façon coulissante dans ces deux orifices, le pion étant en outre enfiché dans ces deux orifices de façon que la tête d'épaulement 72 soit au contact de la face de la came qui est tournée vers l'autre came, et des moyens pour solidariser la tige de pion 71 uniquement avec la biellette 41, 42.

Ces derniers moyens pour solidariser la tige de pion 71 avec uniquement la biellette 41, 42 sont choisis parmi les moyens suivants : une soudure, un brasage, un collage, de préférence par soudure lorsque les matériaux utilisés pour la réalisation des biellettes 41, 42 et des pions 70 le permettent. De préférence donc, ces matériaux sont en acier inoxydable ou analogue.

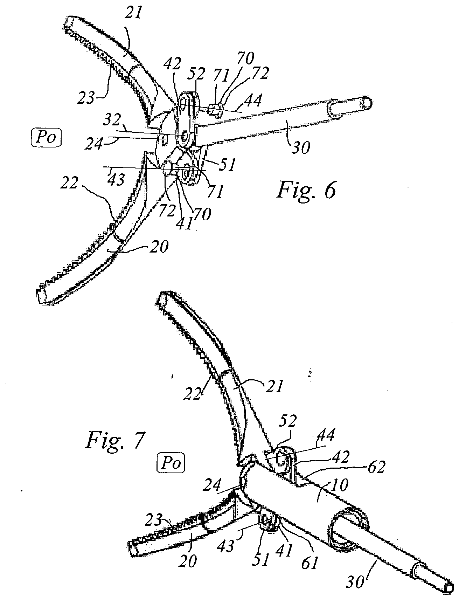

De façon très avantageuse, la chape 10 comporte, dans la paroi de son extrémité distale 13, deux découpes ouvertes 61, 62 agencées de façon à permettre le passage des extrémités liées des deux biellettes 41, 42 et des deux cames 51, 52 lorsque les mors 20, 21 passent de la position fermée Pf à la position ouverte Po, voir par exemple les illustrations des figures 5, 6 et surtout 7.

Lors de son utilisation, la pince doit être introduite dans un tube guide (non illustré). La structure de la pince selon l'invention permet de réaliser un tube guide qui a une percée traversante longitudinale cylindrique d'un diamètre sensiblement égal au diamètre extérieur 2D de la chape 10 et avantageusement très légèrement supérieur, juste nécessaire pour obtenir un glissement de la chape de la pince dans le tube guide.

Sur la figure 2, l'encombrement hors tout transversal des deux mors 20, 21 (défini dans le plan de la figure 2) est illustré en étant inférieur à celui des deux biellettes. Mais ces deux encombrements peuvent être avantageusement sensiblement identiques et donc sensiblement égaux au diamètre extérieur de la chape 10, comme illustré sur les figures 3 et 4.

A la description faite ci-dessus, il apparaît notamment que les biellettes peuvent être d'une largeur maximale, en tout état de cause, pour une même application, plus larges que celles des pinces de coelioscopie de l'art antérieur, ce qui ne peut que conférer à la pince selon l'invention une fiabilité accrue, en évitant notamment une rupture potentielle des biellettes qui absorbent un maximum des efforts lorsque la pince est utilisée en coelioscopie.

**Revendications**

1. Pince de coelioscopie comportant au moins :

    • une chape (10) ayant un diamètre extérieur constant sur toute sa longueur fonctionnelle définie entre son extrémité proximale (12) et son extrémité distale (13) et comprenant une percée traversante cylindrique de révolution (11) définie entre lesdites extrémité proximale et extrémité distale,
    • deux mors (20, 21) à faces de préhension (22, 23),
    • des moyens pour monter en rotation, à l'extrémité distale (13) de ladite percée traversante (11), les deux mors par rapport à ladite chape autour d'un premier axe (24) de façon que ces deux mors soient aptes à prendre toutes positions entre deux positions extrêmes, respectivement une position ouverte (Po) quand les deux faces de préhension respectivement des deux mors font entre elles un angle non nul et une position fermée (Pf) quand les deux faces de préhension respectivement des deux mors font entre elles un angle sensiblement nul **et sont sensiblement au contact**,
    • une tige de commande (30) montée en translation (Tr) dans ladite percée traversante (11) et définie entre une extrémité proximale (35) et une extrémité distale (31),
    • des moyens formant pantographe (40) pour lier l'extrémité distale (31) de ladite tige de commande respectivement aux deux dits mors, lesdits moyens formant pantographe comprenant :

        * deux biellettes (41, 42),
        * des moyens pour monter en rotation les deux dites biellettes, respectivement par une première de leurs deux extrémités, à l'extrémité distale (31) de ladite tige (30), par rapport à la tige de commande et autour d'un deuxième axe (32),
        * deux cames (51, 52) respectivement solidaires des deux mors (20, 21) par une première de leurs deux extrémités,
        * des moyens pour monter en rotation la seconde extrémité de chaque biellette (41, 42) sur la seconde extrémité de chaque came (51, 52) autour respectivement d'un troisième axe (43) et d'un quatrième axe (44), lesdits premier (24), deuxième (32), troisième (43) et quatrième (44) axes étant définis de façon à être parallèles,

    • le diamètre extérieur de ladite chape (10) ayant une valeur égale à 2D,
    • les biellettes (41, 42) étant identiques et formées chacune d'une plaque à section transversale rectangulaire à bout arrondi, la valeur de la largeur de cette plaque étant égale à 2E,
    • la distance séparant les troisième (43) et quatrième (44) axes de rotation quand les deux faces de préhension (22, 23) des mors (20, 21) sont au contact **en position fermée (Pf)** ayant une valeur 2C inférieure à 2D, et
    • la valeur de l'angle que font les axes (45, 46) des deux biellettes (41, 42) quand les deux faces de préhension des mors sont au contact, étant égale à 2A,
les valeurs D, E, C et A sont sensiblement liées par l'équation suivante :

$$D = C + E/\text{cosinus } A,$$

**caractérisée par le fait que** :

• le bord extérieur de la seconde extrémité de chaque biellette comporte une partie sensiblement cylindrique chanfreinée (80) d'un rayon sensiblement égal à celui de la surface extérieure (Se) de ladite chape (10) de façon que, quand les deux mors (20, 21) sont en position fermée (Pf), lesdits moyens formant pantographe (40) soient inscrits dans et sur la surface enveloppante définie par ladite surface extérieure (Se) de ladite chape (10).

2. Pince de coelioscopie selon la revendication 1, **caractérisée par le fait que** les deux cames (51, 52) sont situées entre les deux biellettes (41, 42).

3. Pince de coelioscopie selon la revendication 2, **caractérisée par le fait que** les moyens pour monter en rotation la seconde extrémité d'une biellette (41, 42) sur la seconde extrémité d'une came (51, 52) sont constitués par :

• un pion (70) comportant une tige de pion (71) et une tête d'épaulement (72) solidaire de ladite tige de pion (71),
• deux orifices cylindriques de révolution (73, 74) réalisés de façon coaxiale respectivement dans la biellette et dans la came, la section transversale des dits orifices étant sensiblement complémentaires de la section transversale de ladite tige de pion (71) de façon que cette dernière soit apte à être enfichée de façon coulissante dans ces deux orifices, ledit pion étant enfiché dans ces deux orifices de façon que ladite tête d'épaulement (72) soit au contact de ladite came, et
• des moyens pour solidariser ladite tige de pion (71) avec ladite biellette (41, 42).

4. Pince de coelioscopie selon la revendication 3, **caractérisée par le fait que** les moyens pour solidariser ladite tige de pion (71) avec ladite biellette (41, 42) sont choisis parmi les moyens suivants : une soudure, un brasage, un collage.

5. Pince de coelioscopie selon l'une des revendications précédentes, **caractérisée par le fait que** la chape (10) comporte, dans la paroi de son extrémité distale (13), deux découpes ouvertes (61, 62) agencées de façon à permettre le passage des extrémités liées des deux biellettes (41, 42) et des deux cames (51, 52) lorsque les mors (20, 21) passent de la position fermée (Pf) à la position ouverte (Po).

6. Ensemble **caractérisé par le fait qu'**il comprend une pince de coelioscopie selon au moins l'une des revendications précédentes et un tube guide comportant une percée longitudinale traversante cylindrique d'un diamètre sensiblement égal au diamètre extérieur de ladite chape (10).

**Patentansprüche**

1. Laparoskopiezange, umfassend mindestens:

- ein Querhaupt (10) mit einem konstanten Außendurchmesser auf seiner ganzen funktionalen Länge, die zwischen seinem proximalen Ende (12) und seinem distalen Ende (13) festgelegt ist, und mit einem kreiszylindrischen Durchgangsloch (11), das zwischen dem proximalen Ende und dem distalen Ende festgelegt ist,
- zwei Klemmbacken (20, 21) mit Greifflächen (22, 23),
- Mittel zum drehbaren Befestigen der zwei Klemmbacken in Bezug auf das Querhaupt um eine erste Achse (24) am distalen Ende (13) des Durchgangslochs (11), so dass diese zwei Klemmbacken geeignet sind, alle Positionen zwischen zwei Endpositionen anzunehmen, einer offenen Position (Po), wenn die zwei Klemmflächen der zwei jeweiligen Klemmbacken zueinander einen von null verschiedenen Winkel bilden, bzw. einer geschlossenen Position (Pf), wenn die zwei Greifflächen der zwei jeweiligen Klemmbacken zwischen sich einen Winkel von im Wesentlichen null bilden und im Wesentlichen in Kontakt stehen,
- eine Steuerstange (30), die in dem Durchgangsloch (11) translatorisch (Tr) montiert ist und zwischen einem proximalen Ende (35) und einem distalen Ende (31) definiert ist,
- Mittel, die einen Pantographen (40) bilden, um das distale Ende (31) der Steuerstange jeweils mit den zwei Klemmbacken zu verbinden, wobei die einen Pantographen bildenden Mittel umfassen:

- zwei Kniehebel (41, 42),
- Mittel zum drehbaren Befestigen der zwei Kniehebel jeweils über ein erstes ihrer zwei Enden am distalen Ende (31) der Stange (30) in Bezug auf die Steuerstange und um eine zweite Achse (32),
- zwei Nocken (51, 52), die jeweils mit den zwei Klemmbacken (20, 21) durch ein erstes ihrer zwei Enden fest verbunden sind,
- Mittel zum drehbaren Befestigen des zweiten Endes jedes Kniehebels (41, 42) am zweiten Ende jedes Nockens (51, 52) um eine dritte Achse (43) bzw. eine vierte Achse (44), wobei die erste (24), die zweite (32), die dritte (43) und die vierte (44) Achse parallel definiert sind,

- wobei der Außendurchmesser des Querhaupts (10) einen Wert gleich 2D aufweist,
- die Kniehebel (41, 42) identisch sind und jeweils aus einer Platte mit rechteckigem Querschnitt mit abgerundeten Enden gebildet sind, wobei der Wert der Breite dieser Platte gleich 2E ist,
- der Abstand, der die dritte (43) und die vierte (44) Drehachse trennt, wenn die zwei Greifflächen (22, 23) der Klemmbacken (20, 21) in der geschlossenen Position (Pf) in Kontakt stehen, einen Wert 2C geringer als 2D aufweist, und
- der Wert des Winkels, den die Achsen (45, 46) der zwei Kniehebel (41, 42) bilden, wenn die zwei Greifflächen der Klemmbacken in Kontakt stehen, gleich 2A ist,

wobei die Werte D, E, C und A im Wesentlichen über die folgende Gleichung in Beziehung stehen:

$$D = C + E/\text{cosinus } A$$

**dadurch gekennzeichnet, dass**:
- der äußere Rand des zweiten Endes jedes Kniehebels einen im Wesentlichen zylindrischen, abgeschrägten Abschnitt (80) mit einem Radius umfasst, der im Wesentlichen gleich jenem der äußeren Oberfläche (Se) des Querhaupts (10) ist, so dass dann, wenn die zwei Klemmbacken (20, 21) sich in der geschlossenen Position (Pf) befinden, die einen Pantographen (40) bildenden Mittel in und auf der Hüllfläche einbeschrieben sind, die durch die äußere Oberfläche (Se) des Querhaupts (10) festgelegt ist.

2. Laparoskopiezange nach Anspruch 1, **dadurch gekennzeichnet, dass** die zwei Nocken (51, 52) zwischen den zwei Kniehebeln (41, 42) angeordnet sind.

3. Laparoskopiezange nach Anspruch 2, **dadurch gekennzeichnet, dass** die Mittel zum drehbaren Befestigen des zweiten Endes eines Kniehebels (41, 42) am zweiten Ende eines Nockens (51, 52) gebildet sind durch:

- einen Zapfen (70) mit einem Zapfenschaft (71) und einem Ansatzkopf (72), der mit dem Zapfenschaft (71) fest verbunden ist,
- zwei rotationszylindrische Öffnungen (73, 74), die koaxial im Kniehebel bzw. im Nocken ausbildet sind, wobei der Querschnitt der Öffnungen zum Querschnitt des Zapfenschafts (71) im Wesentlichen komplementär ist, derart dass dieser letztere geeignet ist, verschiebbar in diese zwei Öffnungen eingesteckt zu werden, wobei der Zapfen in diese zwei Öffnungen eingesteckt wird, so dass der Ansatzkopf (72) mit dem Nocken in Kontakt steht, und
- Mittel zum Befestigen des Zapfenschafts (71) an dem Kniehebel (41, 42).

4. Laparoskopiezange nach Anspruch 3, **dadurch gekennzeichnet, dass** die Mittel zum Befestigen des Zapfenschafts (71) an dem Kniehebel (41, 42) aus den folgenden Mitteln ausgewählt sind: einer Verschweißung, einer Hartlötung, einer Verklebung.

5. Laparoskopiezange nach einem der vorangehenden Ansprüche, **dadurch gekennzeichnet, dass** das Querhaupt (10) in der Wand seines distalen Endes (13) zwei offene Schnitte (61, 62) umfasst, die so angeordnet sind, dass sie den Durchlass der Enden, die mit den zwei Kniehebeln (41, 42) und den zwei Nocken (51, 52) verbunden sind, ermöglichen, wenn die Klemmbacken (20, 21) von der geschlossenen Position (Pf) in die offene Position (Po) übergehen.

6. Baugruppe, **dadurch gekennzeichnet, dass** sie eine Laparoskopiezange nach mindestens einem der vorangehenden Ansprüche und ein Führungsrohr mit einem zylindrischen Längsdurchgangsloch mit einem Durchmesser umfasst, der im Wesentlichen gleich dem Außendurchmesser des Querhaupts (10) ist.

**Claims**

1. Laparoscopy forceps comprising at least:

   • a yoke (10) having an outside diameter that is constant over its entire functional length defined between its proximal end (12) and its distal end (13) and having a circularly cylindrical through hole (11) defined between said proximal and distal ends;
   • two jaws (20, 21) having respective grip faces (22, 23);
   • means for mounting the two jaws to the distal end (13) of said through hole (11) to pivot relative to said yoke about a first axis (24) in such a manner that the two jaws are suitable for occupying all positions between two extreme positions, respectively an open position (Po) when the two grip faces of the respective jaws form a non-zero angle between each other, and a closed position (Pf) when the two grip faces of the respective jaws form a substantially zero angle between each other and are substantially in contact;
   • a control rod (30) mounted to move in translation (Tr) in said through hole (11) and defined between a proximal end (35) and a distal end (31); and
   • pantograph-forming means (40) for connecting the distal end (31) of said control rod respectively to both of said jaws, said pantograph-forming means comprising:

      * two links (41, 42);
      * means for mounting said two links via respective first ones of their two ends to the distal end (31) of said rod (30) to pivot relative to the control rod about a second axis (32);
      * two cams (51, 52) secured to respective ones of the two jaws (20, 21) at first ones of their two ends; and
      * means for mounting the second end of each link (41, 42) to the second end of a respective one of the cams (51, 52) to pivot respectively about a third axis (43) and a fourth axis (44), said first, second, third, and fourth axes (24, 32, 43, and 44) being defined so as to parallel,

   • the outside diameter of said yoke (10) having a value equal to 2D;
   • the links (41, 42) being identical, each being formed by a plate of rectangular cross-section with rounded ends, the width of said plate being equal to 2E;
   • the distance between the third and fourth pivot axes (43 and 44) when the two grip faces (22, 23) of the jaws (20, 21) are in contact in closed position (Pf) having a value 2C that is less than 2D; and
   • the angle between the axes (45, 46) of two links (41, 42) when the two grip faces of the jaws are in contact being equal to 2A;
   the values D, E, C, and A being substantially associated by the following equation:

   $$D = C + E/\cos A$$

   **characterized by** the fact that:

      • the outer edge at the second end of each link has a substantially chamfered cylindrical portion (80) of radius substantially equal to the radius of the outer surface (Se) of said yoke (10) so that, when the two jaws (20, 21) are in the closed position (Pf), said pantograph-forming means (40) are inscribed in and against the envelope surface defined by said outer surface (Se) of said yoke (10).

2. Laparoscopy forceps according to claim 1, **characterized by** the fact that the two cams (51, 52) are situated between the two links (41, 42).

3. Laparoscopy forceps according to claim 2, **characterized by** the fact that the means for mounting the second end of a link (41, 42) to pivot relative to the second end of a cam (51, 52) are constituted by:

   - a peg (70) comprising a peg shank (71) and a shoulder-forming head (72) secured to said peg shank (71);
   - two circularly cylindrical orifices (73, 74) made on a common axis respectively in the link and in the cam, the cross-section of said orifices being substantially complementary to the cross-section of said peg shank (71) so that the shank can be engaged slidably in the two orifices, said peg being engaged in the two orifices in such a manner that said shoulder-forming head (72) is in contact with said cam; and
- means for securing said peg shank (71) to said link (41, 42).

4. Laparoscopy forceps according to claim 3, **characterized by** the fact that the means for securing said peg shank (71) to said link (41, 42) are selected from the following means: welding; brazing; adhesive.

5. Laparoscopy forceps according to any preceding claim, **characterized by** the fact that the yoke (10) includes, in its distal end (13), two open-ended slots (61, 62) arranged so as to pass the connected-together ends of the two links (41, 42) and the two cams (51, 52) when the jaws (20, 21) go from the closed position (Pf) to the open position (Po).

6. An assembly, **characterized by** the fact that it comprises laparoscopy forceps according to at least one of the preceding claims and a guide tube having a cylindrical through longitudinal hole of diameter substantially equal to the outside diameter of said yoke (10).

Fig. 1

Fig. 5

Fig. 3

Fig. 4

Fig. 2

Fig. 6

Fig. 7

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5312434 A

- DE 29911011 U1